# EUROPEAN PATENT APPLICATION

(11) **EP 4 844 055 A1**
(43) Date of publication of application: **25.11.2026**
(21) Application number: 25741933.3
(22) Date of filing: 14.01.2025
(51) Int. Cl.: C12N 1/04, C12N 5/071

(54) **HIGH CONCENTRATION CELL PRESERVATION LIQUID AND HIGH CONCENTRATION CELL PRESERVATION METHOD**

(30) Priority: 15.01.2024 JP 2024004162
(71) Applicant: Cellgentech, Inc., Chiba-shi, Chiba 260-0856 (JP)
(72) Inventor: AOYAGI, Yasuyuki, Chiba-shi, Chiba 260-0856 (JP); KAYABA, Atsuko, Chiba-shi, Chiba 260-0856 (JP)
(74) Representative: Grünecker Patent- und Rechtsanwälte PartG mbB
(86) International application number: PCT/JP2025/000910
(87) International publication number: WO 2025/154708

(57) **Abstract**

A novel high-concentration cell preservation solution and a high-concentration cell preservation method are provided.

The cell preservation solution is for preserving cells in a non-frozen state, and contains a basal medium and an additive added to the basal medium. The basal medium is a medium used for cell culture. The additive comprises at least recombinant albumin and ascorbic acid or an ascorbic acid derivative. The cell preservation solution preserves cells at a cell concentration of at least 1 × 10⁷ cells/mL. For example, when cells are preserved in the cell preservation solution at a cell concentration of 1 × 10⁸ cells/mL at 4°C, the cell viability after a lapse of 72 hours is 80% or more.

## Description

### Technical Field

The present invention relates to a high-concentration cell preservation solution and a high-concentration cell preservation method, and more particularly to a high-concentration cell preservation solution and a method for preserving cells in a non-frozen state.

### Background Art

In recent years, regenerative medicine utilizing cells collected from patients has been practiced.

When a cellular product (also referred to as a cell preparation) is directly administered to a patient, it is preferable to use a cellular product prepared at a high cell concentration in order to reduce the burden on the patient. However, preserving cells at such a high cell concentration poses a problem of decreased cell viability.

Therefore, in current cell therapy, a high-concentration cellular product must be administered to a patient immediately after preparation, necessitating that the patient receive treatment with the cellular product at a university hospital or the like equipped with an advanced cell processing facility.

Patent Literature 1 discloses an invention of a solution composition for preserving cells in a non-frozen state; however, it is not contemplated that the solution composition preserves cells at a high cell concentration.

In addition, Patent Literature 2 discloses an invention of a cryopreservation solution for preserving stem cells in a frozen state, and for example, discloses that stem cells are preserved at a cell concentration of 1 × 10⁴ to 1 × 10⁸ cells/mL. However, since it is just a cell preservation solution in which cells are preserved in a frozen state, a thawing operation is separately required, and the residual rate of the number of viable cells decreases at the time of freeze-thawing. Therefore, it is preferable that the cells can be preserved in a non-frozen state.

### Citation List

### Patent Literature

Patent Literature 1: WO 2019/49985 A
Patent Literature 2: JP 2019-154329 A

### Summary of Invention

### Technical Problem

For example, if there is a cell preservation solution capable of preserving cells at a high cell concentration and maintaining cells at a high viability, cell therapy can be performed not only at a university hospital having a cell processing facility but also at a medical institution located away from the cell processing facility. In addition, when introducing a cellular product (cell preparation) in a non-frozen state into the market, it is preferable that the cell preservation solution is capable of preserving cells for 72 hours or more in consideration of traffic conditions, patient circumstances, and the like.

Furthermore, not limited to cell therapy applications, the cell preservation solution as described above can be widely used for research reagents and other applications, which is beneficial.

An object of the present invention is to provide a novel high-concentration cell preservation solution and a high-concentration cell preservation method.

Another object of the present invention is to provide a high-concentration cell preservation solution and a high-concentration cell preservation method capable of preserving cells at a high cell concentration in a non-frozen state and maintaining cells at a high viability.

### Solution to Problem

As a result of intensive studies, the present inventors have revealed that it is possible to produce a cell preservation solution capable of preserving cells at a high cell concentration in a non-frozen state and maintaining a high viability, and have completed the present invention.

Specifically, as a result of intensive studies mainly on a suitable combination of a basal medium and additives added to the basal medium, the present inventors have revealed that it is possible to produce a cell preservation solution capable of preserving cells at a cell concentration of at least 1 × 10⁷ cells/mL even in a state where the cells are not frozen. For example, the present inventors have revealed that when cells are suspended in the cell preservation solution and the cells are preserved at a cell concentration of 1 × 10⁸ cells/mL at 4°C, the cell viability after a lapse of 72 hours is 80% or more. In addition, the present inventors have revealed that the cell preservation solution can be produced with a pharmaceutically transplantable component.

Therefore, according to the high-concentration cell preservation solution of the present invention, the problem is solved by a cell preservation solution for preserving cells in a non-frozen state, containing a basal medium and an additive added to the basal medium, wherein the basal medium is a medium used for cell culture, the additive comprises at least recombinant albumin and ascorbic acid or an ascorbic acid derivative, and the cells are preserved at a cell concentration of at least 1 × 10⁷ cells/mL.

According to the above configuration, it is possible to realize a high-concentration cell preservation solution and a high-concentration cell preservation method capable of preserving cells at a high cell concentration in a non-frozen state and maintaining a high viability.

At this time, it is preferable that the cells are cells derived from a mammal, the recombinant albumin is recombinant human serum albumin, and the cell preservation solution is used for cell transplantation.

In addition, it is preferable that the cells are preserved at a cell concentration of 1 × 10⁸ to 1 × 10⁹ cells/mL.

Further, it is preferable that the cells are human-derived somatic cells or human-derived stem cells, and the basal medium is at least one medium selected from the group consisting of RPMI 1640 and DMEM.

Furthermore, it is preferable that the concentration of the recombinant albumin is 0.1 to 2.0 (w/v)%. Also, it is preferable that the concentration of the ascorbic acid or the ascorbic acid derivative is 0.1 to 10 mg/mL.

Moreover, when the cells are preserved at a cell concentration of 1 × 10⁸ cells/mL at 4°C, the cell viability after a lapse of 72 hours is preferably 80% or more. More preferably, the cell viability after a lapse of 170 hours is 80% or more.

According to the above configuration, it is possible to realize a high-concentration cell preservation solution capable of preserving cells at a higher cell concentration in a non-frozen state and maintaining cells at a higher viability. In particular, when directly transplanting a cell preparation into a patient, it is possible to provide a cell preservation solution capable of reducing the burden on the patient and suitably performing cell therapy.

In addition, according to the high-concentration cell preservation method of the present invention, the problem is also solved by a method of suspending cells in a cell preservation solution and preserving the cells at a cell concentration of at least 1 × 10⁷ cells/mL in a non-frozen state, wherein the cell preservation solution contains a basal medium and an additive added to the basal medium, the basal medium is a medium used for cell culture, and the additive comprises at least recombinant albumin and ascorbic acid or an ascorbic acid derivative.

### Advantageous Effects of Invention

According to the high-concentration cell preservation solution and the high-concentration cell preservation method of the present invention, it is possible to preserve cells at a high cell concentration in a non-frozen state and maintain cells at a high viability.

### Brief Description of Drawings

FIG. 1 is a graph showing changes over time in the viable cell ratio (cell viability) in Test Example 1.
FIG. 2 is a graph showing the results of cell viability evaluation in Test Example 2.
FIG. 3 is a graph showing the results of cytotoxicity evaluation in Test Example 3.
FIG. 4 is a graph showing changes over time in the viable cell ratio in Test Example 4-1.
FIG. 5 is a graph showing changes over time in the viable cell ratio in Test Example 4-2.
FIG. 6 is a graph showing changes over time in the viable cell ratio in Test Example 5.
FIG. 7 is a graph showing changes over time in the viable cell ratio in Test Example 6.
FIG. 8 is a graph showing changes over time in the viable cell ratio in Test Example 7.
FIG. 9 is a graph showing changes over time in the viable cell ratio in Test Example 8.

### Description of Embodiments

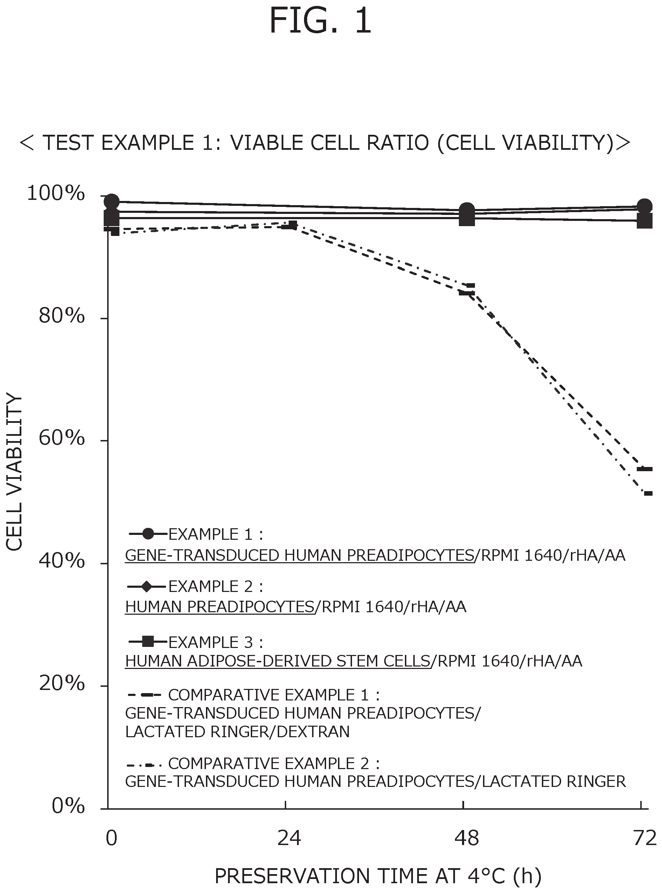

Hereinafter, embodiments of the present invention will be described with reference to FIGS. 1 to 9.

The present embodiment relates to a "high-concentration cell preservation solution" that makes it possible to preserve cells at a high cell concentration in a non-frozen state and to maintain cells at a high viability. The present embodiment also relates to a "high-concentration cell preservation method".

### <Overview of Cell Preservation Solution>

The "cell preservation solution" is a solution composition for preserving cells, and is for preserving cells for performing cell therapy (gene therapy). In the present embodiment, it is a solution composition that preserves cells in a non-frozen state.

The "non-frozen state" is a state of 1 to 20°C, preferably 1 to 15°C, more preferably 1 to 10°C, even more preferably 1 to 5°C, and even more preferably 4°C.

In addition, the cell preservation solution of the present embodiment is not limited to one used as a cellular product, and can be widely used for research reagents and other applications.

The "high-concentration cell preservation solution" is a cell preservation solution for preserving cells at a high cell concentration.

For example, it is one for preserving cells at a cell concentration of at least 1 × 10⁷ cells/mL (1 × 10⁷ cells/mL or more), preferably 5 × 10⁷ cells/mL or more, and more preferably 1 × 10⁸ cells/mL or more. Alternatively, it is one for preserving cells at a cell concentration of 1 × 10⁷ to 1 × 10⁹ cells/mL, more preferably 5 × 10⁷ to 1 × 10⁹ cells/mL, even more preferably 1 × 10⁸ to 1 × 10⁹ cells/mL, and even more preferably 1 × 10⁸ cells/mL.

By preserving (non-frozen preserving) cells at a cell concentration of 1 × 10⁷ cells/mL or more (preferably 1 × 10⁸ cells/mL or more), when directly transplanting a cellular product (non-frozen cellular product) into a patient, the transplantation volume can be reduced, and the burden on the patient can be reduced.

In addition, high cell viability can be maintained by preserving cells at a cell concentration of 1 × 10⁹ cells/mL or less.

Regarding the "high cell viability", it is preferable that when cells are preserved at a cell concentration of 1 × 10⁷ to 1 × 10⁹ cells/mL (preferably 1 × 10⁸ cells/mL) at 4°C, the cell viability after a lapse of 72 hours is 70% or more, preferably 75% or more, more preferably 80% or more, even more preferably 85% or more, and even more preferably 90% or more. Alternatively, it is preferable that when cells are preserved at the above cell concentration at 4°C, the cell viability after a lapse of 96 hours is 70% or more, preferably 75% or more, more preferably 80% or more, even more preferably 85% or more, and even more preferably 90% or more.

Alternatively, it is preferable that when cells are preserved at the above cell concentration at 4°C, the cell viability after a lapse of 120 hours is 70% or more, preferably 75% or more, more preferably 80% or more, even more preferably 85% or more, and even more preferably 90% or more.

Alternatively, it is preferable that when cells are preserved at the above cell concentration at 4°C, the cell viability after a lapse of 170 hours is 70% or more, preferably 75% or more, more preferably 80% or more, even more preferably 85% or more, and even more preferably 90% or more. Alternatively, it is preferable that the cell viability after a lapse of 144 hours or after a lapse of 168 hours is also the same as described above.

The "cell viability" is preferably calculated by the method used in Test Example 1 described later.

In addition, when introducing a cellular product in a non-frozen state into the market, it is preferable that the cell preservation solution is capable of preserving cells for 72 hours or more for the purpose of transporting the cellular product to medical institutions across the country. Furthermore, it is preferable that the cell preservation solution is composed of components that can be directly transplanted into a patient.

The "cells" preserved by the cell preservation solution are not particularly limited, and various cells can be adopted. Examples thereof include somatic cells, stem cells, and the like.

The "somatic cells" include fibroblasts, B lymphocytes, T lymphocytes, neutrophils, erythrocytes, platelets, macrophages, monocytes, bone cells, pericytes, dendritic cells, keratinocytes, adipocytes, epithelial cells, epidermal cells, endothelial cells, vascular endothelial cells, hepatic parenchymal cells, chondrocytes, cumulus cells, nerve cells (neurons), glial cells (oligodendrocytes, microglia, astrocytes), cardiomyocytes, muscle cells, pancreatic beta cells, melanocytes, hematopoietic progenitor cells, and the like. It is preferable that the somatic cells are adipocytes, more preferably preadipocytes (adipocytes that have acquired proliferative potential and can be harvested by primary culture), even more preferably mammal-derived preadipocytes, and even more preferably human-derived preadipocytes. In the case of adipocytes (preadipocytes), there are no administration restrictions or age restrictions upon administration of a cellular product, the risk of tumorigenesis is low, and the cells are suitable for cell therapy. These somatic cells may be those in which a foreign gene intended for gene therapy is incorporated into their genomic DNA.

The "stem cells" include embryonic stem cells (ES cells), embryonic tumor cells, embryonic germ stem cells, induced pluripotent stem cells (iPS cells), neural stem cells, hematopoietic stem cells, mesenchymal stem cells, hepatic stem cells, pancreatic stem cells, muscle stem cells, germline stem cells, intestinal stem cells, cancer stem cells, hair follicle stem cells, and the like. It is preferable that the stem cells are mesenchymal stem cells, and more preferably human-derived mesenchymal stem cells. These stem cells may be those in which a foreign gene intended for gene therapy is incorporated into their genomic DNA.

### <Configuration of Cell Preservation Solution>

The high-concentration cell preservation solution contains a basal medium and an additive added to the basal medium.

Mainly by a combination of the basal medium and the additive, it is possible to produce a cell preservation solution that can preserve cells at a high cell concentration while maintaining a high viability even when the cells are in a non-frozen state.

### (Basal medium)

The "basal medium" is a medium used for cell culture and is preferably a liquid medium. That is, a buffered salt solution such as a conventional Ringer's solution (a lactated Ringer's solution) or a physiological saline solution is not used as the basal medium.

The "basal medium" is preferably at least one culture medium selected from the group consisting of RPMI 1640 (Roswell Park Memorial Institute 1640), DMEM (Dulbecco's Modified Eagle Medium), MEM (Minimum Essential Medium), αMEM, EMEM, and F-12 (Nutrient Mixture F-12 Medium). The basal medium is preferably RPMI 1640 or DMEM, and more preferably RPMI 1640.

It has been confirmed that by adopting these media as the basal medium, since amino acids, vitamins, and the like necessary for cell survival are contained, cells can be preserved at a high cell concentration for a predetermined time. In particular, by adopting RPMI 1640 or DMEM, an appropriate metabolic state is maintained, and cell preservability is improved. In particular, by adopting RPMI 1640, since glutathione having an antioxidant effect is contained, the cell preservability is further improved.

When RPMI 1640 is used as the basal medium, it is preferable that the basal medium is RPMI 1640 (containing L-glutamine).

When DMEM is used as the basal medium, it is preferable that the basal medium is low-glucose-containing DMEM.

In addition, the medium used for the cell culture may be one in which essential components of a medium are contained in a "buffered salt solution". The essential components of a medium are, for example, "amino acids", "vitamins", and "other components".

The "buffered salt solution" includes a Ringer's solution, lactated Ringer's solution, acetated Ringer's solution, a physiological saline solution, Hank's balanced salt solution (HBSS), Dulbecco's phosphate-buffered saline, and the like.

The "amino acids" include L-alanine, L-arginine, L-asparagine, L-aspartic acid, L-cysteine, L-cystine, L-glutamic acid, L-glutamine, glycine, L-histidine, L-hydroxyproline, L-isoleucine, L-lysine, L-methionine, L-serine, L-threonine, L-tryptophan, L-tyrosine and L-valine, L-phenylalanine, L-leucine, and the like.

The "vitamins" include, in addition to ascorbic acid (an ascorbic acid derivative), inositol, biotin, pantothenic acid, choline, folic acid, niacin, pyridoxine, riboflavin, thiamine, vitamin B12, and the like.

The "other components" include sugars, reduced glutathione, a sodium salt, a potassium salt, inorganic salts other than a sodium salt and a potassium salt, antibiotics, serum, fatty acids, and the like.

The "amino acids", "vitamins", and "other additives" described above may be appropriately added as an additive.

The basal medium is preferably prepared to be contained in an amount of 50 (v/v)% or more, preferably 70 (v/v)% or more, and more preferably 80 (v/v)% or more, based on the total amount of the high-concentration cell preservation solution.

The "additive" has a function of maintaining cell viability by being added to the basal medium.

As a main "additive", it is preferable that recombinant albumin and ascorbic acid are added to the basal medium.

### (Recombinant albumin)

Albumin has an antioxidant effect, and roles in the inhibition of aggregation of cells and the transport of nutrient components in the basal medium to cells. Furthermore, adsorption of cells to a storage container can be inhibited, and difficulty in utilizing cells can be suppressed.

Albumin can be extracted from blood, but also contains many unnecessary impurities. Recombinant albumin is an albumin produced by a genetic recombination technique, and has extremely low contents of impurities and foreign factors.

The albumin to be added is preferably recombinant albumin, and it is further preferable that the albumin is recombinant human serum albumin (rHA).

Recombinant albumin is an albumin produced by a genetic recombination technique, and is obtained by removing xeno-derived components.

In place of the recombinant albumin, a recombinant protein may be added to the basal medium. In this case, it is preferable that a protein having a molecular weight of 20,000 to 100,000 (a low-molecular-weight protein) such as albumin, is used.

The recombinant albumin is preferably prepared to be contained in an amount of 0.01 to 20 (w/v)%, preferably 0.01 to 10 (w/v)%, more preferably 0.01 to 5.0 (w/v)%, and even more preferably 0.1 to 2.0 (w/v)%, based on the total amount of the high-concentration cell preservation solution.

When the content is less than 0.01 (w/v)%, a decrease in viability has been confirmed. In addition, when the content exceeds 20 (w/v)%, the viscosity of the high-concentration cell preservation solution is increased, so that the operability is significantly deteriorated.

### (Ascorbic acid and ascorbic acid derivative)

Ascorbic acid has an antioxidant effect and functions to maintain cell survival. An ascorbic acid derivative similarly has an antioxidant effect. It is preferable that ascorbic acid is used.

The ascorbic acid derivative includes sodium ascorbate, sodium ascorbyl phosphate, magnesium ascorbyl phosphate, ascorbyl glucoside, glyceryl ascorbate, bis-glyceryl ascorbate, L-ascorbyl palmitate, ascorbyl tetrahexyldecanoate, ascorbic acid 2-phosphate, and the like.

It has been confirmed that by adding these additives to the basal medium, cells can be preserved at a high concentration for a predetermined time. In particular, by adding both recombinant albumin and ascorbic acid, cells can be preserved at a higher cell concentration for a predetermined time.

The ascorbic acid (or ascorbic acid derivative) is preferably prepared to be contained in an amount of 0.1 to 10 mg/mL, preferably 0.1 to 5 mg/mL, more preferably 0.1 to 2 mg/mL, and even more preferably 0.1 to 1 mg/mL based on the total amount of the high-concentration cell preservation solution.

It has been confirmed that when ascorbic acid is less than 0.1 mg/mL or exceeds 10 mg/mL, the cell viability decreases.

### (Other additives)

As other "additives", a sodium salt, a potassium salt, inorganic salts other than a sodium salt and a potassium salt, amino acids, vitamins, antibiotics, serum, fatty acids, sugars, and the like may be appropriately added to the basal medium.

Examples of the amino acids include L-alanine, L-arginine, L-asparagine, L-aspartic acid, L-cysteine, L-cystine, L-glutamic acid, L-glutamine, glycine, L-histidine, L-hydroxyproline, L-isoleucine, L-lysine, L-methionine, L-serine, L-threonine, L-tryptophan, L-tyrosine, L-valine, L-phenylalanine, L-leucine, and the like.

Examples of the vitamins include, in addition to the ascorbic acid, biotin, pantothenic acid, choline, folic acid, niacin, pyridoxine, riboflavin, thiamine, vitamin B12, and the like.

The high-concentration cell suspension obtained by suspending cells with the cell preservation solution (or high-concentration cell preservation solution) is preferably preserved in an arbitrary volume of 0.05 mL or more, preferably 0.05 to 500 mL, more preferably 0.05 to 100 mL, and even more preferably 0.05 mL to 50 mL.

The storage container used for preserving cells in a non-frozen state is not particularly limited, and a general-purpose syringe, bag, tube, vial, ampoule, or the like capable of securing these volumes is preferably used.

In addition, it is preferable that a cellular product is aseptically contained in these storage containers. Thereby, even a medical institution or a research facility that does not have an advanced sterile facility can use the cellular product for cell transplantation as it is.

### <High Concentration Cell Preservation Method>

The high-concentration cell preservation method is a method of suspending cells in a cell preservation solution containing the basal medium and the additive, and preserving the cells at a cell concentration of at least 1 × 10⁷ cells/mL in a non-frozen state.

### <Method for Producing High Concentration Cell Suspension Using Cell Preservation Solution>

As a method for producing a high-concentration cell suspension, for example, in the case of producing a "high-concentration cell suspension" for preserving "LCAT gene-transduced human preadipocytes", the following steps are mainly performed.

In the present production method, a "high-concentration cell suspension" can be produced by performing a first step of preparing human preadipocytes into which an LCAT gene is to be transduced, a second step of producing LCAT gene-transduced human preadipocytes, a third step of preparing a cell preservation solution, and a fourth step of recovering, washing, and concentrating the cells and preserving the concentrated cells in a storage container.

More details are as described in Examples described later.

### Examples

Hereinafter, examples of the present invention will be described in detail. Note that the present invention is not limited to these examples.

Using the high-concentration cell preservation solution of the present invention, five types of cells (LCAT gene-transduced human preadipocytes, human preadipocytes, human adipose tissue-derived mesenchymal stem cells, FVIII gene-transduced human preadipocytes, human bone marrow-derived mesenchymal stem cells) were preserved at a high concentration at 4°C, and the cell viability after a lapse of a certain period of time was verified.

For the cell preservation solution, the "basal medium" was an RPMI 1640 medium or a DMEM medium, and the "additive" was recombinant human serum albumin (rHA) and ascorbic acid (AA).

As a comparative example, a commercially available product composed mainly of Ringer's solution was used.

### <Example 1>

As in the following steps (1-1) to (1-4), a high-concentration cell preservation solution was prepared, and a high-concentration cell suspension obtained by suspending "LCAT gene-transduced human preadipocytes" in the cell preservation solution was prepared and preserved.

### Step (1-1) Preparation of human preadipocytes into which an LCAT gene is to be transduced

Adipose tissue (10 g) obtained from a healthy human was dispensed into 50 mL centrifuge tubes in 1 g aliquots, and 3 mL of HBSS (manufactured by Sigma-Aldrich Co. LLC) containing 2 mg/mL collagenase (manufactured by Nordmark Pharma GmbH), 40 µg/mL gentamicin (manufactured by Takata Pharmaceutical Co., Ltd.), and 4 µg/mL vancomycin (manufactured by Pfizer Inc.) was added to each tube, followed by shaking at 37°C for 1 hour.

Next, 10 mL of "DMEM/Nutrient Mixture F-12 Ham (manufactured by Sigma-Aldrich Co. LLC)" supplemented with 20% FBS (manufactured by NICHIREI BIOSCIENCES INC.), 40 µg/mL gentamicin, and 4 µg/mL vancomycin-hereinafter referred to as "DMEM-HAM/20% FBS (GM-VCM)"-was added thereto, and the mixture was stirred and then centrifuged at 400 × g for 1 minute to remove a solution containing a precipitated fraction.

Furthermore, 10 mL of DMEM-HAM/20% FBS (GM-VCM) was added thereto, and the mixture was stirred, followed by centrifugation at 400 × g for 1 minute, and a series of operations of removing a solution containing a precipitated fraction was performed twice to obtain a supernatant fraction containing human preadipocytes.

The supernatant fraction containing human preadipocytes thus obtained was filtered through a 440 µm mesh, and then washing was performed by adding 10 mL of DMEM-HAM/20% FBS (GM-VCM) to the original 50 mL centrifuge tubes, and the washing solution was also filtered.

The filtrate thus obtained was added to a 150 cm² flask previously kept warm at 37°C and filled with DMEM-HAM/20% FBS (GM-VCM). DMEM-HAM/20% FBS (GM-VCM) was further added thereto so that air bubbles remained in the flask as little as possible, and then the flask was sealed.

The flask was placed in a 37°C, 5% CO₂ incubator so that the bottom surface (culture surface) of the flask was a ceiling surface, and ceiling culture was performed for 7 days. After 7 days, the culture solution in the flask was removed, and the culture surface was washed with D-PBS, followed by trypsinization to recover human preadipocytes (4.5 × 10⁷ cells).

### Step (1-2) Preparation of LCAT gene-transduced human preadipocytes

For gene transduction, a retroviral vector was prepared as follows.

An LCAT gene cloned from a cDNA library of a human hepatoma cell line was inserted into a pDON-AI vector plasmid (manufactured by Takara Bio Inc.). A Kozak sequence was added upstream of the 5'-terminal start codon (ATG) of the inserted LCAT gene, and the 3'-terminal stop codon was modified and a polyA signal was removed, thereby producing a retroviral vector plasmid carrying the human LCAT gene.

From this plasmid, a retroviral vector solution carrying the human LCAT gene that can be transduced into human cells was obtained using a packaging system (manufactured by Takara Bio Inc.).

Six days after the start of the ceiling culture in step (1-1) (the day before the recovery of human preadipocytes), 45 mL of a 20 µg/mL RetroNectin (manufactured by Takara Bio Inc.) solution diluted with D-PBS was added to a 225 cm² flask for floating cells, followed by allowing the mixture to stand at 4°C overnight.

Next, after removing the RetroNectin solution, washing was performed once with D-PBS to obtain a RetroNectin-coated flask. The retroviral vector solution carrying the human LCAT gene was diluted to 5 × 10⁸ RNA copies/mL with DMEM-HAM/20% FBS (GM) containing 20% FBS and 40 µg/mL gentamicin, and then 45 mL thereof was added to the RetroNectin-coated flask, followed by allowing the flask to stand at 32°C for 5 hours. After 5 hours, the vector solution was removed to obtain a viral vector-coated flask.

The human preadipocytes recovered in step (1-1) were seeded into the viral vector-coated flasks at a density of 2.25 × 10⁶ cells per 225 cm² flask, and gene transduction was initiated. Two days after the gene transduction, the medium was replaced with a cell culture medium "MSF-BM/MSF-supplement A (manufactured by Shimadzu Diagnostics Corporation)" containing 40 µg/mL gentamicin. After another two days, the cells in the flasks were recovered to obtain LCAT gene-transduced human preadipocytes.

Until the start of the test, the cells were precipitated by centrifugation at 300 × g at 4°C for 5 minutes, suspended in a cell cryopreservation solution (CELLBANKER 1: manufactured by Nippon Zenyaku Kogyo Co., Ltd.), and cryopreserved at -80°C.

### Step (1-3) Preparation of cell preservation solution

To 500 mL of an RPMI 1640 (containing L-glutamine) medium (manufactured by NACALAI TESQUE, INC.) as a "basal medium", 10 mL of Recombumin Elite (manufactured by Albumedix) as "recombinant human serum albumin" was added and mixed.

Furthermore, 1 mL of vitamin C injection "FUSO" (500 mg, manufactured by Fuso Pharmaceutical Industries, Ltd.) as "ascorbic acid" was added thereto and mixed, followed by sterile filtration to obtain a cell preservation solution (a preservation solution before suspending cells).

By preparing the solution as described above, the albumin concentration was adjusted to 0.2 (w/v)% and the ascorbic acid concentration was adjusted to 0.5 mg/mL. The same applied to the concentrations in Examples 2 and 3.

### Step (1-4) Recovery, washing, concentration and preservation of cells

The LCAT gene-transduced human preadipocytes cryopreserved in the step (1-2) were thawed and proliferated in a 37°C, 5% CO₂ incubator using a Nunc EasyFill Cell Factory (manufactured by Thermo Fisher Scientific).

The culture solution was removed, and the culture surface was washed with D-PBS, followed by trypsinization. After the cells were recovered using the cell preservation solution prepared in the step (1-3), the mixture was centrifuged at 3,000 × g at 4°C for 3 minutes to remove the supernatant. Furthermore, a series of operations of suspending the cells in the cell preservation solution, centrifuging the suspension at 3,000 × g at 4°C for 3 minutes, and removing the supernatant was performed three times to wash the cells. After measuring the cell concentration, centrifugation was performed at 3,000 × g and 4°C for 5 minutes, and the cells were suspended in the cell preservation solution so as to be a concentration of 1 × 10⁸ cells/mL. The cell suspension was dispensed into a storage container, and preservation was started at 4°C.

Through the steps (1-1) to (1-4), a high-concentration cell suspension of Example 1, in which the "LCAT gene-transduced human preadipocytes" were suspended in the high-concentration cell preservation solution was obtained.

### <Example 2>

As described below, a high-concentration cell preservation solution was prepared, and a high-concentration cell suspension in which "human preadipocytes" into which no gene was transduced were suspended in the high-concentration cell preservation solution was prepared and preserved.

Human preadipocytes were recovered from adipose tissue obtained from a healthy human according to the method of step (1-1) in Example 1. Until the start of the test, the cells were suspended in a cell cryopreservation solution (CELLBANKER 1) and cryopreserved at -80°C.

A cell preservation solution was prepared according to the method of step (1-3) in Example 1, and recovery, washing, concentration, and preservation of the cells were performed according to the method of step (1-4).

Through the above, a high-concentration cell suspension of Example 2 in which "human preadipocytes" were suspended in the high-concentration cell preservation solution was obtained.

### <Example 3>

As described below, a high-concentration cell preservation solution was prepared, and a high-concentration cell suspension in which "human adipose tissue-derived mesenchymal stem cells" were suspended in the high-concentration cell preservation solution was prepared and preserved.

Human adipose tissue-derived mesenchymal stem cells (manufactured by Thermo Fisher Scientific) were cultured, and the cells were proliferated. Until the start of the test, the cells were suspended in a cell cryopreservation solution (CELLBANKER 1) and cryopreserved at -80°C.

A cell preservation solution was prepared according to the method of step (1-3) in Example 1, and recovery, washing, concentration, and preservation of the cells were performed according to the method of step (1-4).

Through the above, the high-concentration cell suspension of Example 3, in which "human adipose tissue-derived mesenchymal stem cells (human adipose-derived stem cells)" were suspended in the high-concentration cell preservation solution, was obtained.

### <Comparative Examples 1 and 2>

A commercially available dextran 40 and trehalose-containing lactated Ringer's solution as a cell suspension preservation solution was used as the cell preservation solution of Comparative Example 1.

In addition, a commercially available trehalose-containing lactated Ringer's solution as a cell washing preservation solution was used as the cell preservation solution of Comparative Example 2.

Specifically, preparation was performed as in the following steps (2-1) to (2-4).

### Step (2-1) Preparation of human preadipocytes

Following the method of step (1-1) in Example 1, in place of the HBSS containing 2 mg/mL collagenase, 40 µg/mL gentamicin, and 4 µg/mL vancomycin, an HBSS containing 2 mg/mL collagenase and 400 µg/mL vancomycin was used.

In addition, human preadipocytes were obtained using DMEM-HAM/20% FBS (GM) in place of DMEM-HAM/20% FBS (GM-VCM).

### Step (2-2) Preparation of LCAT gene-transduced human preadipocytes

According to the method of step (1-2) in Example 1, LCAT gene-transduced human preadipocytes were produced using MesenPRO RS Medium (manufactured by Thermo Fisher Scientific) in place of MSF-BM/MSF-supplement A.

Until the start of the test, the cells were suspended in a cell cryopreservation solution (CELLBANKER 1) and cryopreserved at -80°C.

### Step (2-3) Preparation of cell washing solution for comparison

To 500 mL of Ringer's solution (manufactured by Otsuka Pharmaceutical Factory, Inc.) as a "cell washing solution" in place of the "basal medium", 10 mL of 25% donated albumin (manufactured by Japan Blood Products Organization) as an "additive" was added and mixed. By performing filtration, a cell washing solution for comparison was obtained.

### Step (2-4) Recovery, washing, concentration and preservation of cells

According to the method of step (1-4) in Example 1, recovery and washing of the cells were performed using the cell washing solution prepared in the step (2-3), and the cells were suspended so as to be a concentration of 0.5 × 10⁸ cells/mL, in each of Comparative Example 1 and Comparative Example 2, followed by starting preservation at 4°C.

Through steps (2-1) to (2-4), the high-concentration cell suspensions of Comparative Examples 1 and 2, in which "LCAT gene-transduced human preadipocytes" were suspended in a commercially available cell preservation solution composed mainly of a Ringer's solution, were obtained.

### <Test Example 1: Calculation of viable cell ratio by cell morphology (different cell types)>

For the high-concentration cell suspensions of Examples 1, 2, and 3, and Comparative Examples 1 and 2, evaluation of the viable cell ratio based on the morphology of the cells immediately after completion of preservation was performed.

At 0 hours, 48 hours, and 72 hours from the start of preservation at 4°C, each high-concentration cell suspension (Examples: 1 × 10⁸ cells/mL, Comparative Examples: 0.5 × 10⁸ cells/mL) in each storage container was extracted, and adjusted to a cell suspension of 5 × 10⁵ cells/mL through serial dilution with each cell culture medium.

The "total cell concentration" and "dead cell concentration" of these cell suspensions were measured using a NucleoCounter NC-100 (manufactured by Chemometec). The "dead cell concentration" was calculated based on the number of cells stained with propidium iodide. The "viable cell ratio (%)" was calculated based on both measured values.

Note that the "viable cell ratio (%)" corresponds to the "cell viability (%)".

### (Results and discussion of Test Example 1)

FIG. 1 shows a summary of changes over time in the viable cell ratio (cell viability) in each of Examples and Comparative Examples. Note that the recombinant human serum albumin was denoted as "rHA", and the ascorbic acid was denoted as "AA".

From the results of Test Example 1, the high-concentration cell suspensions (1 × 10⁸ cells/mL) of Examples 1, 2, and 3 showed a high cell viability even after a lapse of 72 hours from the start of preservation without depending on the type of cells. Specifically, it was found that when cells were preserved at 4°C with the high-concentration cell preservation solution, the cell viability after a lapse of 72 hours was 80% or more, more specifically 85% or more, even more specifically 90% or more, and even more specifically 95% or more (Example 1: 98.3%, Example 2: 97.8%, Example 3: 95.9%).

Note that a plurality of the above tests were performed, and it was confirmed that similar test results were obtained.

In the high-concentration cell suspensions of Comparative Examples 1 and 2, a decrease in cell viability was observed. Specifically, it was found that when the cells were preserved at 4°C with a commercially available cell preservation solution composed mainly of Ringer's solution, the cell viability after a lapse of 72 hours was less than 60% (Comparative Example 1: 55.4%, Comparative Example 2: 51.5%).

It has been confirmed that when the cell concentration is low (for example, lower than 1 × 10⁷ cells/mL), even a cell preservation solution composed mainly of a Ringer's solution as in Comparative Examples 1 and 2 can preserve cells. On the other hand, it was shown that when the cell concentration is high (for example, 1 × 10⁸ cells/mL), it is difficult to preserve cells with a composition using a Ringer's solution or the like.

### <Test Example 2: Cell Viability evaluation by measurement of intracellular dehydrogenase activity>

Test Example 1 verifies only the morphology of the cells. In order to confirm whether cells were appropriately preserved in a state of maintaining their function, cells after completion of preservation were seeded in a culture vessel, and evaluation of cell viability was performed by measuring metabolic activity after 20 hours.

For the high-concentration cell suspensions of Examples 1, 2, and 3, and Comparative Examples 1 and 2, at 0 hours, 48 hours, and 72 hours from the start of preservation at 4°C, each cell suspension in each storage container was extracted, adjusted to a cell suspension of 4 × 10⁴ cells/mL through serial dilution with each cell culture medium, and seeded at 100 µL per well in a 96-well plate. In addition, wells to which only a medium containing no cells was added were prepared.

These were cultured in a 37°C, 5% CO₂ incubator for 20 hours, followed by performing a test according to the attached protocol of Cell Counting Kit-8 (manufactured by DOJINDO LABORATORIES) to obtain an optical density value (OD value) by absorbance measurement at a wavelength of 450 nm.

A value obtained by subtracting the OD value of only the medium from the OD value of each test sample was calculated, and a relative value (an optical density ratio) when the value of 0-hour preservation was 1 was calculated.

### (Results and discussion of Test Example 2)

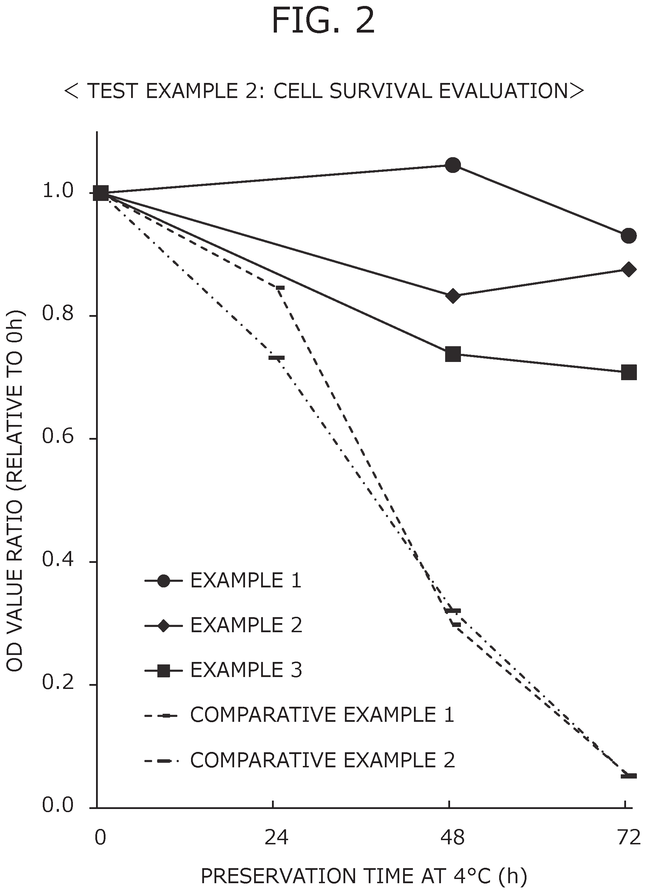

FIG. 2 shows a summary of changes over time in the OD value ratio in each of Examples and Comparative Examples.

From the results of Test Example 2, it was found that unlike the high-concentration cell suspensions of Comparative Examples 1 and 2, the high-concentration cell suspensions of Examples 1, 2, and 3 had high metabolic activity (showed a high OD value ratio) even after a lapse of 72 hours from the start of preservation, indicating that the survival of cells was maintained.

More specifically, in Examples 1, 2, and 3, it was found that when the cells were preserved at 4°C in the high-concentration cell preservation solution, the OD value ratio to that of the 0-hour preservation after a lapse of 72 hours was 0.5 or more, more specifically 0.6 or more, and even more specifically 0.7 or more (Example 1: 0.931, Example 2: 0.876, Example 3: 0.708).

### <Test Example 3: Cytotoxicity evaluation by detection of lactate dehydrogenase (LDH) in culture solution>

In order to evaluate damage to cells preserved in a high-concentration cell preservation solution, the amount of free LDH induced by cell membrane damage was measured.

For the high-concentration cell suspensions of Examples 1, 2, and 3, at 0 hours, 48 hours, and 72 hours from the start of preservation at 4°C, the extracted cells were adjusted to 4 × 10⁴ cells/mL through serial dilution with each cell culture medium, and were seeded at 1000 µL per well of 2 wells of a 12-well plate. In addition, 2 wells to which only a medium containing no cells was added were prepared.

These were cultured in a 37°C, 5% CO₂ incubator for 20 hours, followed by adding a Lysis Buffer attached to an LDH cytotoxicity assay kit (manufactured by NACALAI TESQUE, INC.) to 1 well of 2 wells of each test sample, and culturing was performed in a 37°C, 5% CO₂ incubator for 30 minutes.

After 30 minutes, the culture solution of each test sample was recovered, followed by centrifugation at 5,000 rpm at 4°C for 5 minutes. The supernatant after the centrifugation was recovered and cryopreserved at -80°C until analysis. A test was carried out according to the attached protocol of the LDH cytotoxicity assay kit to obtain an OD value by absorbance measurement at a wavelength of 492 nm. A value obtained by subtracting the OD value of only the medium from the OD value of each test sample was calculated, and a relative value to the sample to which Lysis Buffer was added was calculated.

### (Results and discussion of Test Example 3)

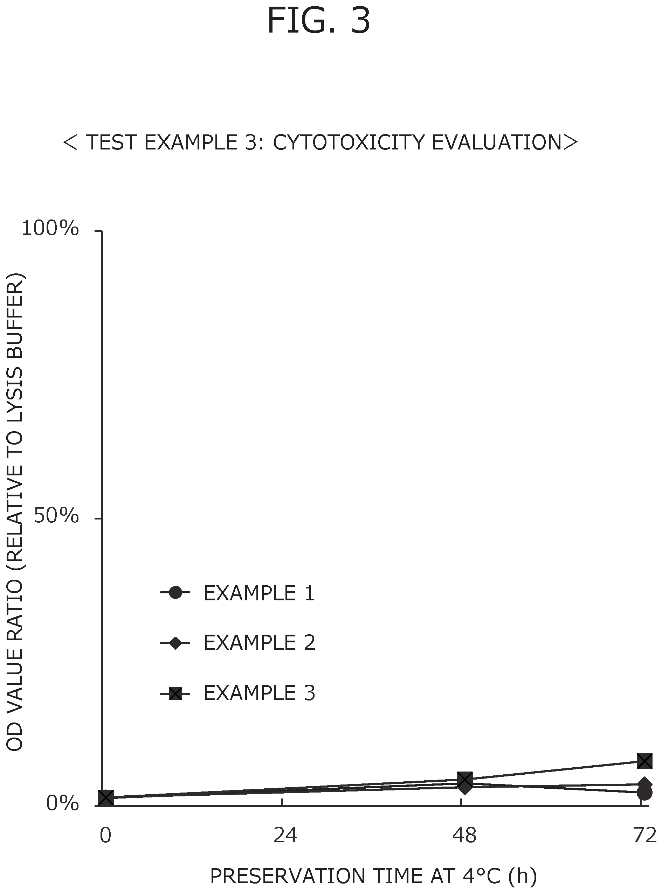

FIG. 3 shows a summary of changes over time in the relative value of the OD values in each of Examples.

From the results of Test Example 3, it was found that in the high concentration cell suspensions of Examples 1, 2, and 3, the relative value of the OD value was low even after a lapse of 72 hours from the start of preservation, indicating that cytotoxicity was not observed.

More specifically, in Examples 1, 2, and 3, it was found that when the cells were preserved at 4°C in the high-concentration cell preservation solution, the relative value to the sample to which the Lysis Buffer was added after a lapse of 72 hours was 10% or less, and even more specifically 8% or less (Example 1: 2.33%, Example 2: 3.75%, Example 3: 7.80%).

### <Example 4>

By preparing the suspension as in Example 2, the high-concentration cell suspension of Example 4, in which "human preadipocytes" were suspended in the high-concentration cell preservation solution was obtained.

For the high-concentration cell suspension, the "cell concentration" was 1 × 10⁸ cells/mL, the "basal medium" was an RPMI 1640 (containing L-glutamine) medium, and the "additive" was recombinant human serum albumin (rHA) and ascorbic acid (AA).

In addition, the concentrations of albumin were 0.1 (w/v)%, 0.2 (w/v)%, and 2.0 (w/v)%, and the concentrations of ascorbic acid were 0.1 mg/mL, 0.5 mg/mL, 1 mg/mL, 2 mg/mL, 5 mg/mL, and 10 mg/mL.

### <Test Example 4: Calculation of viable cell ratio by cell morphology (additives at different concentrations)>

In the same manner as in Test Example 1, evaluation of the viable cell ratio was performed for the high-concentration cell suspension of Example 4 having different concentrations of albumin and ascorbic acid.

### (Results and discussion of Test Example 4)

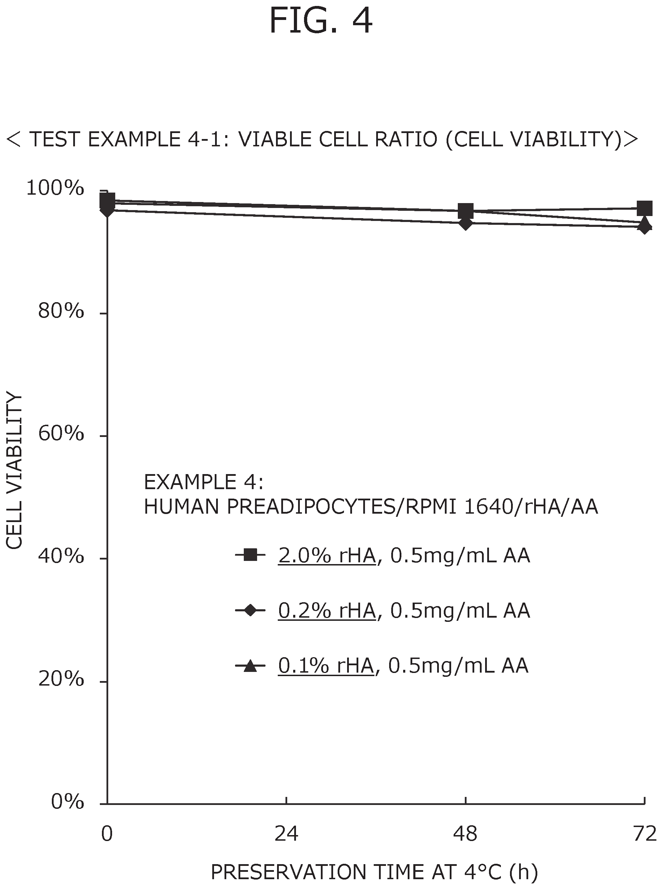

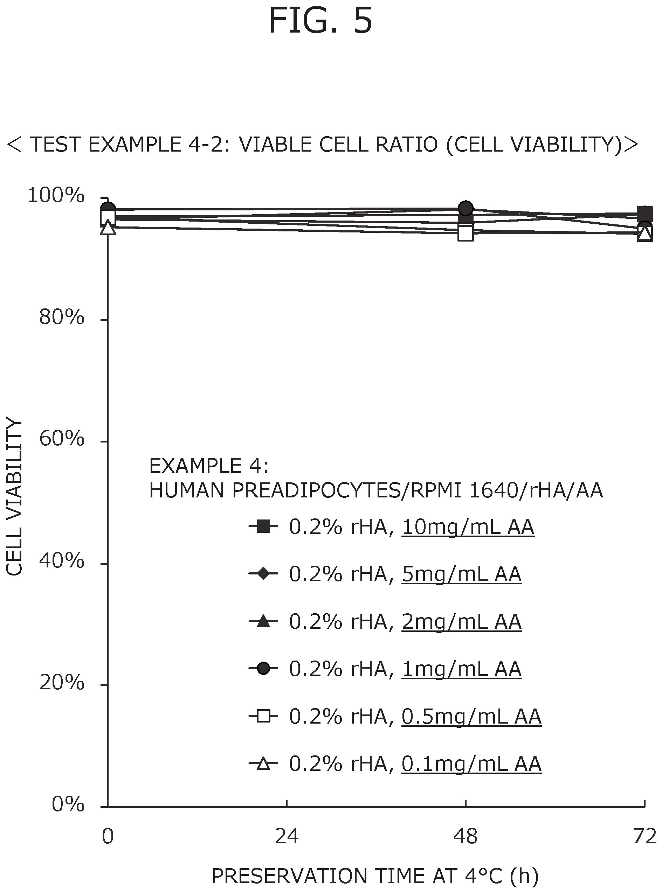

FIGS. 4 and 5 show a summary of changes over time in the viable cell ratio (cell viability) in Example 4 having different albumin concentrations and ascorbic acid concentrations. Note that the recombinant human serum albumin was denoted as "rHA", and the ascorbic acid was denoted as "AA".

From the results of Test Example 4-1, the high concentration cell suspension (1 × 10⁸ cells/mL) of Example 4 when the albumin concentration (rHA concentration) was 0.1 to 2.0 (w/v)% showed a high cell viability even after a lapse of 72 hours from the start of preservation. Specifically, it was found that when cells were preserved at 4°C, the cell viability after a lapse of 72 hours was 90% or more (rHA concentration 0.1 (w/v)%: 94.8%, rHA concentration 0.2 (w/v)%: 94.1%, rHA concentration 2.0 (w/v)%: 97.1%).

Also, from the results of Test Example 4-2, the high-concentration cell suspension of Example 4 when the ascorbic acid concentration (AA concentration) was 0.1 mg/mL to 10 mg/mL showed high viability even after a lapse of 72 hours from the start of preservation. Specifically, it was found that when cells were preserved at 4°C, the cell viability after a lapse of 72 hours was 90% or more (AA concentration 0.1 mg/mL: 94.4%, AA concentration 0.5 mg/mL: 94.1%, AA concentration 1 mg/mL: 95.0%, AA concentration 2 mg/mL: 96.7%, AA concentration 5 mg/mL: 97.5%, AA concentration 10 mg/mL: 97.4%).

Note that a plurality of the above tests were performed, and it was confirmed that similar test results were obtained.

In addition, for the high-concentration cell suspension of Example 4, a cell viability evaluation similar to that in Test Example 2 was performed to confirm the maintenance of cell viability. Also, a cytotoxicity evaluation similar to that in Test Example 3 was performed to confirm that cytotoxicity was not observed.

### <Examples 5 and 6>

As in Example 1, the high-concentration cell suspensions of Examples 5 and 6, in which "LCAT gene-transduced human preadipocytes" were suspended in the high-concentration cell preservation solution, were obtained.

For Example 5, the "cell concentration" was 1 × 10⁸ cells/mL, the "basal medium" was RPMI 1640 (containing L-glutamine), and the "additive" was recombinant human serum albumin (rHA), ascorbic acid (AA), and sodium pyruvate (SP). The albumin concentration was 0.5 (w/v)%, the ascorbic acid concentration was 5 mg/mL, and the sodium pyruvate concentration was 1 mM.

For Example 6, the "cell concentration" was 1 × 10⁸ cells/mL, the "basal medium" was DMEM (low glucose) (manufactured by NACALAI TESQUE, INC.), and the "additive" was recombinant human serum albumin (rHA) and ascorbic acid (AA). The albumin concentration was 0.5 (w/v)%, and the ascorbic acid concentration was 5 mg/mL.

Note that since the RPMI 1640 of Example 5 did not contain sodium pyruvate, it was decided to separately add sodium pyruvate. Since the DMEM of Example 6 contained sodium pyruvate, equivalent composition conditions were achieved.

### <Test Example 5: Calculation of viable cell ratio by cell morphology (different basal media)>

In the same manner as in Test Example 1, evaluation of the viable cell ratio (cell viability) was performed for the high-concentration cell suspensions of Examples 5 and 6 having different basal media.

### (Results and discussion of Test Example 5)

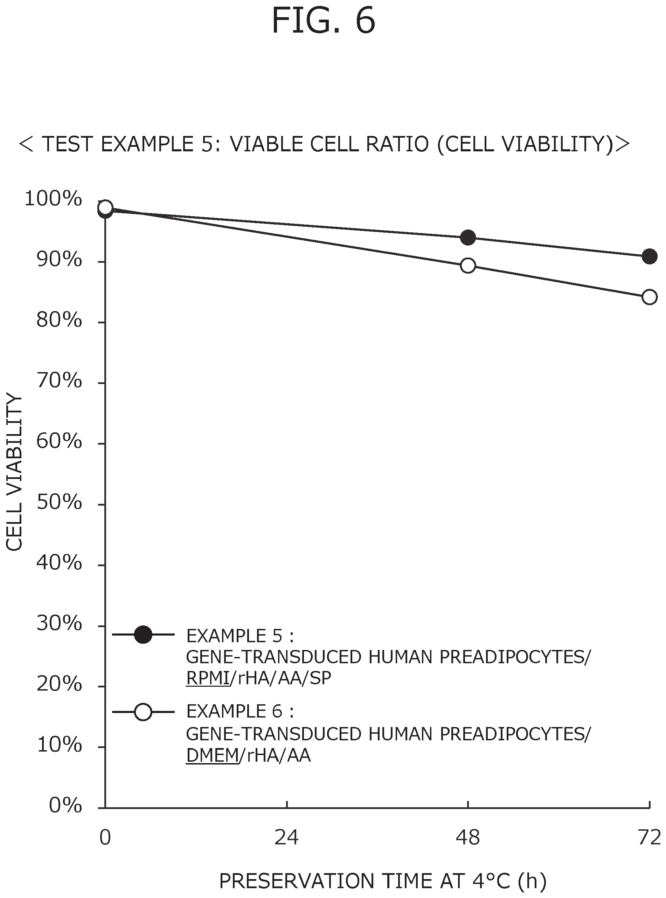

FIG. 6 shows a summary of changes over time in the viable cell ratio (cell viability) in Examples 5 and 6. Note that the recombinant human serum albumin was denoted as "rHA", the ascorbic acid was denoted as "AA", and the sodium pyruvate was denoted as "SP".

From the results of Test Example 5, the high-concentration cell suspensions (1 × 10⁸ cells/mL) of Examples 5 and 6 having "RPMI 1640" or "DMEM" as the basal medium showed high cell viability even after a lapse of 72 hours from the start of preservation. Specifically, it was shown that when cells were preserved at 4°C, the cell viability after a lapse of 72 hours was 80% or more (Example 5: 90.9%, Example 6: 84.2%).

Furthermore, it was shown that the cell viability when using "RPMI 1640" as the basal medium was higher than that when using "DMEM". Note that a plurality of the above tests were performed, and it was confirmed that similar test results were obtained.

Additionally, for the high-concentration cell suspensions of Examples 5 and 6, a cell viability evaluation similar to that in Test Example 2 was performed to confirm the maintenance of cell viability.

### <Example 7>

A high-concentration cell preservation solution was prepared as in the following steps (7-1) to (7-3), and a high-concentration cell suspension obtained by suspending "FVIII gene-transduced human preadipocytes" in the cell preservation solution was prepared and preserved.

### Step (7-1) Preparation of human preadipocytes into which an FVIII gene is to be transduced

Human preadipocytes were recovered from adipose tissue obtained from a healthy human according to the method of step (1-1) in Example 1. Until the start of the test, the cells were suspended in a cell cryopreservation solution (CELLBANKER 1) and cryopreserved at -80°C.

### Step (7-2) Preparation of FVIII gene-transduced human preadipocytes

For gene transduction, a lentiviral vector was prepared as follows.

First, an "FVIII variant" was produced in which the number of Asn residues modified with an N-type sugar chain in the B domain of the human FVIII gene was set to five from the N-terminal side, the function of a furin recognition site was deleted, and the Pro residue at position 758 was replaced with a Thr residue. A base sequence obtained by adding the signal peptide to the "FVIII variant" was inserted into a pLVSIN vector plasmid (manufactured by Takara Bio Inc.) to produce a lentiviral vector plasmid carrying the human FVIII gene.

From this plasmid, a lentiviral vector solution carrying the human FVIII gene that can be transduced into human cells was obtained using a packaging system (manufactured by Takara Bio Inc.).

The preadipocytes frozen in step (7-1) were thawed, followed by culturing in a 150 cm² flask in a 37°C, 5% CO₂ incubator overnight.

On the next day, the culture solution was removed, and the culture surface was washed with D-PBS, followed by trypsinization to recover preadipocytes. The lentiviral vector solution carrying the human FVIII gene obtained in this step was adjusted with MSF-BM/MSF-Supplement A so as to be a concentration of 1 × 10⁹ RNA copies/mL, contain 100 µg/mL protamine sulfate, and have a cell concentration of 2.5 × 10⁴ cells/mL. Then, 5 mL thereof was seeded in a 25 cm² flask to initiate gene transduction, and the medium was replaced on the next day.

On the 2nd day and the 7th day after the first gene transduction, the same gene transduction operation was performed (three times in total) to obtain "FVIII gene-transduced human preadipocytes".

Until the start of the test, the cells were precipitated by centrifugation at 300 × g at 4°C for 5 minutes, suspended in a cell cryopreservation solution (CELLBANKER 1), and cryopreserved at -80°C.

### Step (7-3) Recovery, washing, concentration and preservation of cells

According to the method of step (1-3) in Example 1, a cell preservation solution with "RPMI 1640 (containing L-glutamine) medium" as the basal medium was prepared, and recovery, washing, concentration, and preservation of the cells were performed according to the method of step (1-4).

Through the above, a high-concentration cell suspension of Example 7, in which "FVIII gene-transduced human preadipocytes" were suspended in the high-concentration cell preservation solution having an "RPMI 1640 (containing L-glutamine) medium" as a basal medium, was obtained.

### <Example 8>

As described below, a high-concentration cell preservation solution was prepared, and a high-concentration cell suspension in which "human bone marrow-derived mesenchymal stem cells" were suspended in the high-concentration cell preservation solution was prepared and preserved.

Human bone marrow-derived mesenchymal stem cells (manufactured by PromoCell) were cultured, and the cells were proliferated. Until the start of the test, the cells were suspended in a cell cryopreservation solution (CELLBANKER 1) and cryopreserved at -80°C.

A cell preservation solution was prepared according to the method of step (1-3) in Example 1, and recovery, washing, concentration, and preservation of the cells were performed according to the method of step (1-4).

Through the above, the high-concentration cell suspension of Example 8, in which "human bone marrow-derived mesenchymal stem cells" were suspended in a high-concentration cell preservation solution having an "RPMI 1640 medium" as the basal medium, was obtained.

### <Test Example 6: Calculation of viable cell ratio by cell morphology (different cell types)>

In the same manner as in Test Example 1, evaluation of viable cell ratio (cell viability) was performed for the high-concentration cell suspensions of Examples 1, 2, 7, and 8, and Comparative Examples 1 and 2 having different cell types.

Note that for Examples 1, 2, and 7, the test was performed on a plurality of samples.

### (Results and discussion of Test Example 6)

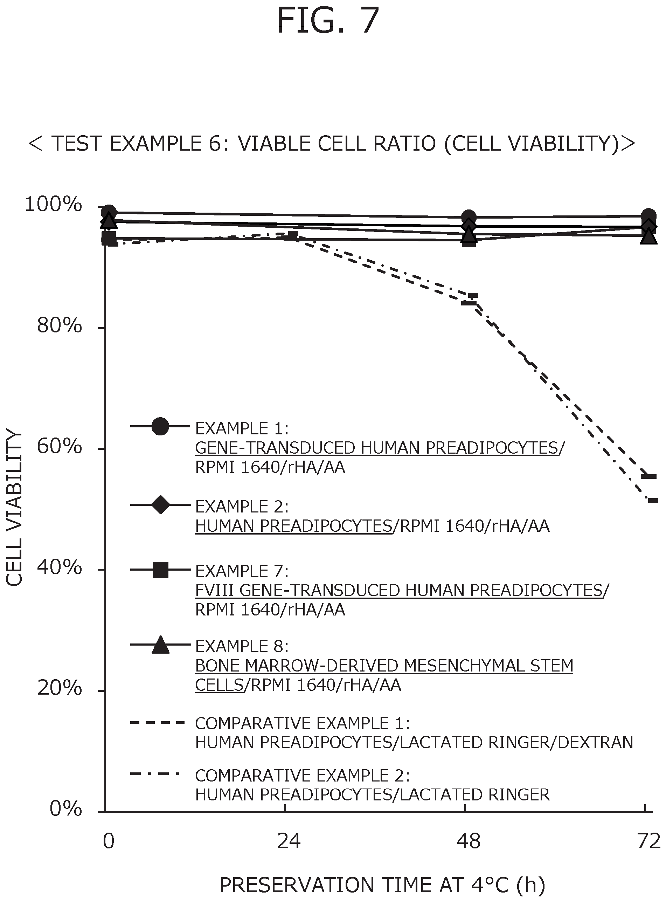

FIG. 7 shows a summary of changes over time in the viable cell ratio (cell viability) in Examples and Comparative Examples having different cell types. Note that the recombinant human serum albumin was denoted as "rHA", and the ascorbic acid was denoted as "AA".

From the results of Test Example 6, the high-concentration cell suspensions (1 × 10⁸ cells/mL) of Examples 1, 2, 7, and 8 showed a high cell viability even after a lapse of 72 hours from the start of preservation without depending on the type of cells. Specifically, it was found that when the cells were preserved at 4°C with the high-concentration cell preservation solution, the cell viability after a lapse of 72 hours was 80% or more, more specifically 85% or more, even more specifically 90% or more, and even more specifically 95% or more (Example 1: 98.4% (n=3), Example 2: 96.7% (n=4), Example 7: 96.7% (n=3), Example 8: 95.3% (n=1)).

Furthermore, for Examples 7 and 8, a cell viability evaluation and a cytotoxicity evaluation were performed together in the same manner as in Test Examples 2 and 3, and it was confirmed that they had high metabolic activity even after a lapse of 72 hours from the start of preservation, and that no cytotoxicity was observed.

### <Examples 9 and 10>

As in Example 2, the high-concentration cell suspensions of Examples 9 and 10, in which "human preadipocytes" into which no gene was transduced were suspended in a high-concentration cell preservation solution were obtained.

For Example 9, the "cell concentration" was 1 × 10⁸ cells/mL, the "basal medium" was RPMI 1640, and the "additive" was recombinant human serum albumin (rHA) and ascorbic acid (AA). The albumin concentration was 0.2 (w/v)%, and the ascorbic acid concentration was 0.5 mg/mL.

For Example 10, the "cell concentration" was 1 × 10⁸ cells/mL, the "basal medium" was DMEM (low glucose), and the "additive" was recombinant human serum albumin (rHA) and ascorbic acid (AA). The albumin concentration was 0.2 (w/v)%, and the ascorbic acid concentration was 0.5 mg/mL.

### <Examples 11 and 12>

As in Example 1, the high-concentration cell suspensions of Examples 11 and 12, in which "LCAT gene-transduced human preadipocytes" were suspended in the high-concentration cell preservation solution, were obtained.

For Example 11, the "cell concentration" was 1 × 10⁸ cells/mL, the "basal medium" was RPMI 1640 (containing L-glutamine), and the "additive" was recombinant human serum albumin (rHA) and ascorbic acid (AA). The albumin concentration was 0.2 (w/v)%, and the ascorbic acid concentration was 0.5 mg/mL.

For Example 12, the "cell concentration" was 1 × 10⁸ cells/mL, the "basal medium" was DMEM (low glucose), and the "additive" was recombinant human serum albumin (rHA) and ascorbic acid (AA). The albumin concentration was 0.2 (w/v)%, and the ascorbic acid concentration was 0.5 mg/mL.

Note that although the RPMI 1640 of Examples 9 and 11 did not contain sodium pyruvate, since it was confirmed that the presence or absence of sodium pyruvate had almost no effect on cell viability, cell survival maintenance, and cytotoxicity, it was decided not to add sodium pyruvate.

### <Test Example 7: Calculation of viable cell ratio by cell morphology (different basal media)>

In the same manner as in Test Example 5, evaluation of the viable cell ratio (cell viability) was performed for the high-concentration cell suspensions of Examples 9, 10, 11, and 12 having different basal media.

### (Results and discussion of Test Example 7)

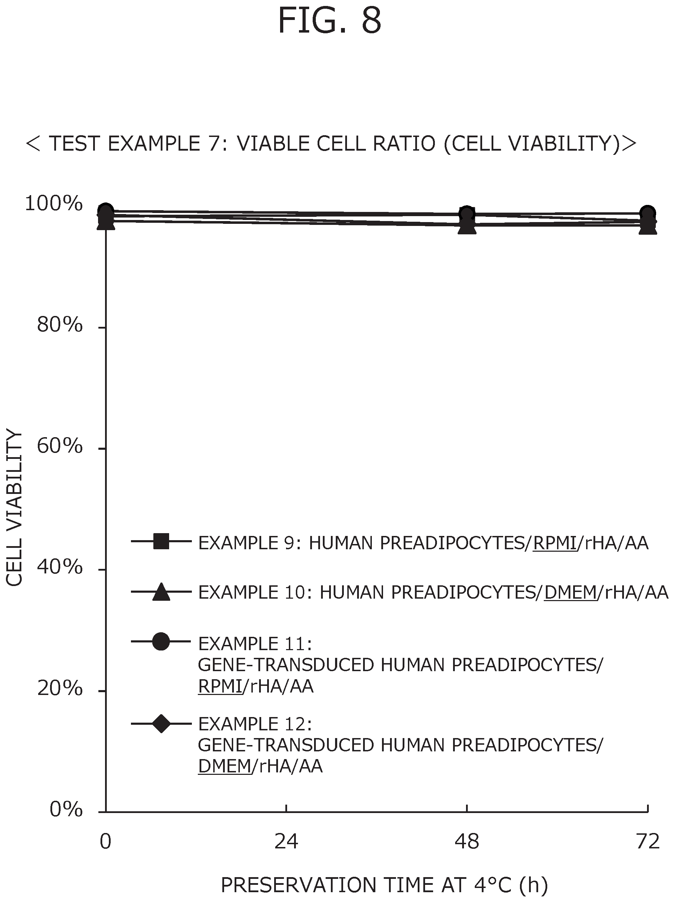

FIG. 8 shows a summary of changes over time in the viable cell ratio (cell viability) in Examples 9, 10, 11, and 12. Note that the recombinant human serum albumin was denoted as "rHA", and the ascorbic acid was denoted as "AA".

From the results of Test Example 7, the high-concentration cell preservation suspensions (1 × 10⁸ cells/mL) of Examples 9, 10, 11, and 12 having "RPMI 1640" or "DMEM" as the basal medium, and "LCAT gene-transduced human preadipocytes" or "human preadipocytes" as the preserved cells showed a high cell viability even after a lapse of 72 hours from the start of preservation. Specifically, it was shown that when cells were preserved at 4°C, the cell viability after a lapse of 72 hours was 96% or more (Example 9: 97.6%, Example 10: 96.8%, Example 11: 98.8%, Example 12: 97.4%).

In addition, it was shown that the cell viability when using "RPMI 1640" as the basal medium was higher than that when using "DMEM" (the cell viability of Examples 9 and 11 was higher than that of Examples 10 and 12, respectively). Note that a plurality of the above tests were performed, and it was confirmed that similar test results were obtained.

Furthermore, in Examples 9 and 11, it was confirmed that the cell viability was higher when sodium pyruvate was not intentionally added.

Additionally, for Examples 9 and 10, a cell viability evaluation and a cytotoxicity evaluation were performed in the same manner as in Test Examples 2 and 3 to confirm the maintenance of cell viability, and it was confirmed that no cytotoxicity was observed.

### <Test Example 8: Calculation of viable cell ratio by cell morphology (long-term survival ratio)>

In the same manner as in Test Example 1, evaluation of the long-term viability was performed for the high-concentration cell suspensions of Examples 9 and 10 with different basal media.

### (Results and discussion of Test Example 8)

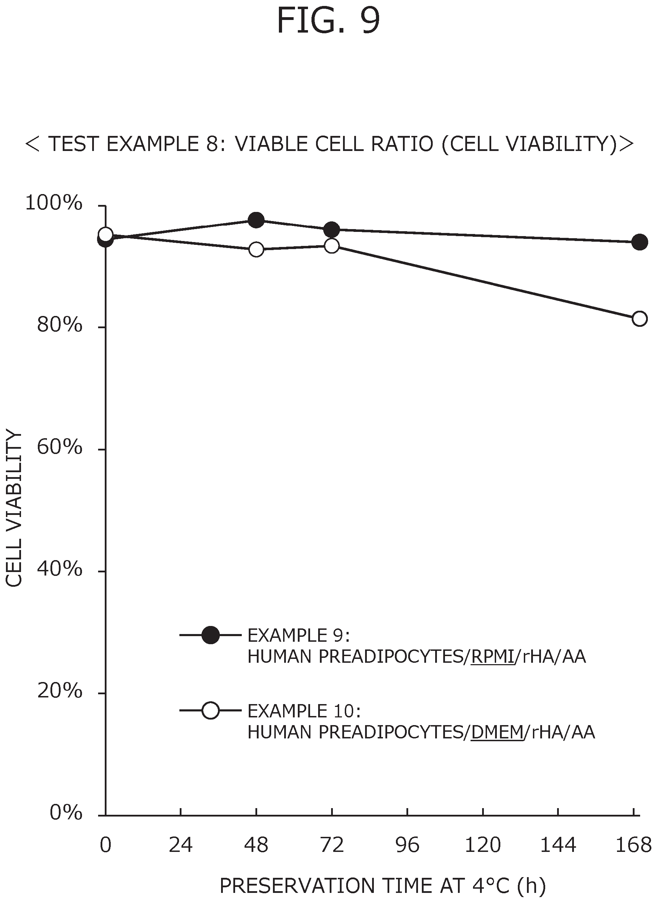

FIG. 9 shows a summary of changes over time in the viable cell ratio (cell viability) in Examples 9 and 10.

From the results of Test Example 8, the high-concentration cell suspensions (1 × 10⁸ cells/mL) of Examples 9 and 10 showed high cell viability even after a lapse of 170 hours from the start of preservation. Specifically, it was found that when cells were preserved at 4°C with the high-concentration cell preservation solution, the cell viability after a lapse of 170 hours was 80% or more. In particular, in the case of Example 9 having the "RPMI 1640 medium" as a basal medium, it was found that the cell viability after a lapse of 170 hours was 90% or more (Example 9: 94.0%, Example 10: 81.4%).

Note that a plurality of the above tests were performed, and it was confirmed that similar test results were obtained.

### <Supplementary Note>

Examples 1 to 12 are summarized as follows.
Example 1: LCAT gene-transduced human preadipocytes, RPMI 1640
Example 2: Human preadipocytes, RPMI 1640
Example 3: Human adipose-derived stem cells, RPMI 1640
Example 4: Human preadipocytes, RPMI 1640, different concentrations of additive
Example 5: LCAT gene-transduced human preadipocytes, RPMI 1640, sodium pyruvate
Example 6: LCAT gene-transduced human preadipocytes, DMEM
Example 7: FVIII gene-transduced human preadipocytes, RPMI 1640
Example 8: Human bone marrow-derived mesenchymal stem cells, RPMI 1640
Example 9: Human preadipocytes, RPMI 1640
Example 10: Human preadipocytes, DMEM
Example 11: LCAT gene-transduced human preadipocytes, RPMI 1640
Example 12: LCAT gene-transduced human preadipocytes, DMEM

## Claims

1. A high-concentration cell preservation solution for preserving cells in a non-frozen state, comprising:
a basal medium, and
an additive added to the basal medium, wherein
the basal medium is a medium used for cell culture,
the additive comprises at least recombinant albumin and ascorbic acid or an ascorbic acid derivative, and
the cells are preserved at a cell concentration of at least 1 × 10⁷ cells/mL.

2. The high-concentration cell preservation solution according to claim 1, wherein
the cells are cells derived from a mammal,
the recombinant albumin is a recombinant human serum albumin, and
the high-concentration cell preservation solution is used for cell transplantation.

3. The high-concentration cell preservation solution according to claim 1, wherein the cells are preserved at a cell concentration of 1 × 10⁸ to 1 × 10⁹ cells/mL.

4. The high-concentration cell preservation solution according to claim 1, wherein
the cells are human-derived somatic cells or human-derived stem cells, and
the basal medium is at least one medium selected from the group consisting of RPMI 1640 and DMEM.

5. The high-concentration cell preservation solution according to claim 1, wherein the concentration of the recombinant albumin is 0.1 to 2.0 (w/v)%.

6. The high-concentration cell preservation solution according to claim 1, wherein the concentration of the ascorbic acid or the ascorbic acid derivative is 0.1 to 10 mg/mL.

7. The high-concentration cell preservation solution according to any one of claims 1 to 6, wherein when the cells are preserved at a cell concentration of 1 × 10⁸ cells/mL at 4°C, the cell viability after a lapse of 72 hours is 80% or more.

8. The high-concentration cell preservation solution according to any one of claims 1 to 6, wherein when the cells are preserved at a cell concentration of 1 × 10⁸ cells/mL at 4°C, the cell viability after a lapse of 170 hours is 80% or more.

9. A high-concentration cell preservation method of suspending cells in a cell preservation solution and preserving the cells at a cell concentration of at least 1 × 10⁷ cells/mL in a non-frozen state, wherein
the cell preservation solution contains a basal medium and an additive added to the basal medium,
the basal medium is a medium used for cell culture, and
the additive comprises at least recombinant albumin and ascorbic acid or an ascorbic acid derivative.
